(19) Europäisches Patentamt
European Patent Office
Office européen des brevets

(11) Veröffentlichungsnummer: **0 157 739**
**B1**

(12) # EUROPÄISCHE PATENTSCHRIFT

(45) Veröffentlichungstag der Patentschrift: **12.08.87**

(21) Anmeldenummer: **85810128.0**

(22) Anmeldetag: **25.03.85**

(51) Int. Cl.[4]: **C 07 C 29/40**, C 07 C 31/36, C 07 C 33/42, C 07 C 33/46, C 07 C 69/96

(54) **Substituierte 1-Propanole, Verfahren zu deren Herstellung, Chlorameisensäureester und deren Verwendung.**

(30) Priorität: **29.03.84 CH 1582/84**

(43) Veröffentlichungstag der Anmeldung: **09.10.85 Patentblatt 85/41**

(45) Bekanntmachung des Hinweises auf die Patenterteilung: **12.08.87 Patentblatt 87/33**

(84) Benannte Vertragsstaaten: **BE CH DE FR GB IT LI NL**

(56) Entgegenhaltungen:
**DE-A-1 816 282**
**DE-A-2 557 162**
**FR-A-1 496 633**
**US-A-3 732 274**

(73) Patentinhaber: **CIBA- GEIGY AG, Klybeckstrasse 141, CH- 4002 Basel (CH)**

(72) Erfinder: **Lang, Robert Werner, Dr., Hagenbachweg 10, CH- 4133 Pratteln (CH)**

EP 0 157 739 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft in α-Stellung substituierte und teilhalogenierte 1-Propanole, ein Verfahren zur Herstellung von teilhalogenierten 1-Propanolen, deren Chlorameisensäureester und deren Verwendung als O- oder N-Schutzgruppen.

In der FR-PS 1 496 633 ist die Herstellung von 3,3,3-Trifluor-2,2-dichlor-1-propanol durch die Umsetzung von Formaldehyd, Fluorwasserstoffsäure und 1,1-Dichlor-2,2-difluorethylen im Autoklaven bei erhöhter Temperatur beschrieben. Die Verbindung wird nur in sehr geringen Mengen (weniger als 2 %) gebildet. Das 1,1-Dichlor-2,2-difluorethylen ist teuer und zudem muss wegen dessen Flüchtigkeit im Autoklaven gearbeitet werden, was unwirtschaftlich ist.

Gegenstand vorliegender Erfindung sind teilhalogenierte α-substituierte l-Propanole der Formel 1

$$F_3C\text{-}CCl_2\text{-}\overset{\displaystyle R}{\overset{|}{C}}H\text{-}OH \qquad (I),$$

worin R ein äliphatischer oder aromatischer Kohlenwasserstoffrest oder ein über ein C-Atom gebundener aliphatischer oder aromatischer heterocyclischer Rest ist der gegebenenfalls durch $C_1$-$C_{18}$-Alkyl, $C_2$-$C_{18}$-Alkenyl, $C_2$-$C_{18}$-Alkinyl, $C_6$-$C_{18}$-Aryl, $C_7$-$C_{18}$-Aralkyl, $C_7$-$C_{18}$-Alkaryl, $C_8$-$C_{18}$-Alkaralkyl, $C_1$-$C_{18}$-Alkoxy, $C_1$-$C_{18}$-Alkylthio, $C_6$-$C_{18}$-Aryloxy, $C_7$-$C_{18}$-Aralkoxy, $C_7$-$C_{18}$-Alkaryloxy, $C_8$-$C_{18}$-Alkaraloxy, $C_7$-$C_{18}$-Aryloxyalkyl, $C_7$-$C_{18}$-Alkoxyaryl, $C_3$-$C_{12}$-Cycloalkoxy, $C_3$-$C_{12}$-Cycloalkenyl, $C_3$-$C_{12}$-Cycloalkenyloxy, -COOH, $-CONH_2$, F, -CN, -OH, -SH, $-NH_2$, $-COOR^1$, $-CNHR^1$, $-CONR^1R^2$, $-NHR^1$, $-NR^1R^2$, worin $R^1$ $C_1$-$C_{18}$-Alkyl, Cyclohexyl, Phenyl oder Benzyl ist und $R^2$ unabhängig die gleiche Bedeutung wie $R^1$ hat, für R in der Bedeutung als aromatischer Rest auch durch Br oder Cl substituiert ist, und wobei Rauch p-Chlorphenoxymethyl sein kann oder in der Bedeutung als aliphatischer Rest gegebenenfalls durch -CO- unterbrochen ist.

R als Kohlenwasserstoffrest enthält bevorzugt 1 bis 30, besonders 1 bis 20 und insbesondere 1 bis 12 C-Atome, R als heterocyclischer Rest enthält bevorzugt 1 bis 3 Heteroatome aus der Gruppe O, N und S im Ring. Der Ring enthält bevorzugt 4 bis 8, besonders 5 oder 6 Ringglieder. Es können gleiche oder verschiedene Heteroatome im Ring enthalten sein. Der Rest R ist unsubstituiert oder mit vorzugsweise 1 bis 3 wie zuvor definierten Resten substituiert.

R ist bevorzugt als aliphatischer Kohlenwasserstoffrest Alkyl, Alkenyl, Alkinyl, Cycloalkyl oder Cycloalkenyl, als aromatischer Kohlenwasserstoffrest Aryl, Aralkenyl, Aralkinyl oder Aralkyl, als heterocyclisch-aliphatischer Rest ein 4- bis 6-gliedriger, 1 oder 2 Heteroatome aus der Gruppe O, S und N enthaltender heterocyclischer Rest und als aromatischer-heterocyclischer Rest ein 5- oder 6-gliedriger, 1 oder 2 Heteroatome aus der Gruppe O, N und S enthaltender heterocyclischer Rest, die wie zuvor definiert substituiert sein können. Das Alkyl, Alkenyl und Alkinyl kann linear oder verzweigt sein. Beispiele für Alkyl sind Methyl, Ethyl, n-Propyl, iso-Propyl, n-, iso- und tertiär-Butyl, Pentyl, Hexyl, Octyl, 2-Ethylhexyl, Nonyl, Decyl, Undecyl, Dodecyl, Tetradecyl und Eicosyl. Beispiele für Alkenyl sind Vinyl Allyl 1- oder 2-Methylvinyl, Styryl, Butenyl, Pentenyl, Hexenyl, Octenyl, Decenyl und Dodecenyl.

Beispiele für Alkinyl sind Ethinyl, Propargyl, Methylethinyl, Phenylethinyl, Butinyl, Pentinyl, Hexinyl, Octinyl, Decinyl und Dodecinyl.

Das Cycloalkyl und Cycloalkenyl kann 3 bis 12, vorzugsweise 3 bis 8 und insbesondere 3 bis 6 Ringkohlenstoffatome enthalten. Beispiele sind Cyclopropyl, Cyclobutyl, Cyclopentyl, Cyclohexyl, Cycloheptyl, Cyclooctyl, Cyclononyl, Cyclodecyl, Cyclododecyl, Cyclopropenyl, Cyclobutenyl, Cyclopentadienyl, Cyclohexenyl, Cyclohexadienyl, Cyclooctenyl, Cyclooctadienyl, Bicyclo-2,2,2-octenyl, Bicyclo-2,2,1-heptenyl.

Der aromatische Kohlenwasserstoffrest kann ein- oder mehrkernig und kondensiert sein. Bevorzugt sind Aryl, Aralkenyl, Aralkinyl oder Aralkyl mit insbesondere 6 bis 18, bzw. 7 bis 18, bzw. 8 bis 18 C-Atomen Das Aryl ist bevorzugt Phenyl oder Naphthyl. Beispiele sind Phenyl, Naphthyl, Benzyl, Phenylethyl, 2-Phenylpropyl, Naphthylmethyl, Dihydronaphthalin, Indan, Inden, Fluoren, Phenanthren.

R als aliphatisch-heterocyclischer Rest kann 3 bis 7, vorzugsweise 4 bis 6 Ringglieder und je nach Ringgrösse 1 bis 3, bevorzugt 1 oder 2 Heteroatome aus der Gruppe O, S und N enthalten. Beispiele für Heteroringe von denen sich der Rest R ableitet, sind: Oxiran, Oxetan, Oxolan, Oxolen, Oxan, Dioxan, Aziridin, Azetidin, Azetin, Pyrrolidin, Pyrrolin, Tetrahydrothiophen, 2,3-Dihydroindol, Dihydrocumaron, Dihydrothionaphthen, Pyrazolidin, Imidazolidin, Oxazolidin, Thiazolidin, Triazolidin, Oxadiazolidin, Morpholin, Piperidin, Tetrahydrochinolin.

R als heteroaromatischer Rest enthält bevorzugt 1 bis 3, besonders 1 bis 2 Heteroatome aus der Gruppe O, S und N und stellt bevorzugt einen 5- oder 6-gliedrigen Ring dar. Ferner kommen auch kondensierte Ringsysteme in Frage. Beispiel für Heteroaromaten, von den sich R ableiten kann sind: Pyrrol, Furan, Thiophen, Pyridin, Pyran, Pyrazol, Imidazol, Benzinidazol, Triazin, Oxazol, Thiazol, Pyrimidin, Pyrazin, Chinolin, Chromen, Purin und Xanthen.

Der Rest R kann ein- oder mehrfach mit gleichen oder verschiedenen wie zuvor definierten Resten substituiert sein, bevorzugt ein- bis dreifach. Geeignete Substituenten für R sind solche, die unter den angegebenen Reaktionsbedingungen nicht durch das eingesetzte Metall reduziert werden.

Die aliphatischen Reste R können im Rahmen der im Anspruch 1 gegebenen Definition durch Heteroatome, -CO-, -C(O)O- -CONH- oder $-CONR^1$- unterbrochen und/oder durch -OH, -SH, F, $-NH_2$, $-NHR^1$, $-NR^1R^2$, -CN, -COOH, $-COOR^1$, $-CONH_2$, $-CONHR^1$ oder $-CONR^1R^2$ substituiert sein.

Im Substituent ist das Aryl bevorzugt Phenyl. Bevorzugt sind $C_1$-$C_{12}$-Alkyl, $C_2$-$C_{12}$-Alkenyl, Phenyl, Benzyl, $C_7$-$C_{14}$-Alkylphenyl, $C_8$-$C_{14}$-Alkylbenzyl, $C_1$-$C_2$-Alkoxy, Phenoxy, Benzyloxy, $C_7$-$C_{12}$-Alkylphenoxy, $C_8$-$C_{14}$-Alkylbenzyloxy, $C_7$-$C_{12}$-Phenoxyalkyl, $C_7$-$C_{14}$ -Alkoxyphenyl, Cyclopentyl, Cyclohexyl, -COOH, $-CONH_2$, $-COOR^1$ mit $R^1$ gleich $C_1$-$C_{12}$-Alkyl; CN, OH, SH, $NH_2$, F, Cl, Br.

Einige Beispiele sind: Methyl, Ethyl, Propyl, Butyl, Vinyl, Allyl, Ethinyl, Propargyl, Phenyl, Benzyl, Methylbenzyl, Methylphenyl, Methoxy, Ethoxy, Propoxy, Butoxy, Methylthio, Phenoxy, Benzyloxy, Methylphenoxy, Methylbenzyloxy, Phenoxymethyl, Methoxyphenyl, Cyclopentyl, Cyclohexyl, Cyclohexenyl, Cyclohexyloxy, Methoxycarbonyl, Methylamino, Dimethylamino, Methylaminocarbonyl. Die Reste können im Rahmen der im Anspruch 1 gegebenen Definition durch die Heteroatome O, S und N unterbrochen sein. Beispiele für solche Reste R sind: Alkoxyalkyl wie Methoxymethyl oder Methoxyethyl, Alkylaminoalkyl wie Methylaminomethyl oder Dimethylaminoethyl, Phenoxyphenyl, Methoxycarbonylmethyl oder Ethoxycarbonylethyl. Die Substituenten können ihrerseits im Rahmen der im Anpruch 1 gegebenen Definition substituiert sein.

Die erfindungsgemässen Verbindungen können nach einem neuen Verfahren durch die Umsetzung von 1,1,1-Trifluor-2,2,2-trichlorethan mit Aldehyden hergestellt werden. Ein weiterer Gegenstand verliegender Erfindung ist ein Verfahren zur Herstellung von teilhalogenierten, α-substituierten 1-Propanolen der Formel II

$$F_3CCCl_2\text{-}\underset{}{\overset{R^3}{\overset{|}{C}}}H\text{-}OH \qquad (II),$$

worin $R^3$ für ein Wasserstoffatom steht oder die gleiche Bedeutung wie R in Formel I hat, das dadurch gekennzeichnet ist, dass man 1,1,1-Trifluor-2,2,2-trichloräthan der Formel III

$$F_3C\text{-}CCl_3 \qquad (III),$$

mit einem Aldehyd der Formel IV

$$R^3\text{-}CHO \qquad (IV),$$

worin $R^3$ die für Formel II angegebene Bedeutung hat, in Gegenwart eines Metalles, Mischungen von Metallen oder Metallegierungen aus der Gruppe Eisen, Kupfer, Zinn, Cadmium und Zink, oder der Metallverbindung Zinn(II)chlorid, und in Gegenwart eines inerten Lösungsmittels umsetzt, das Reaktionsgemisch nach Beendigung der Reaktion mit einer Säure behandelt und danach das l-Propanol der Formel II in an sich bekannter Weise isoliert.

Die Reaktion wird vorzugsweise bei Raumtemperatur durchgeführt, wobei die Reaktionszeiten von Stunden bis Tage betragen können. Wird die Reaktion unter Einwirkung von Ultraschall z. B. im Bereich von 22 - 38 kHz vorgenommen, so lassen sich die Reaktionszeiten erheblich reduzieren.

Die Verbindung der Formel III ist kommerziell erhältlich und die Aldehyde der Formel IV sind bekannt oder können nach bekannten Verfahren hergestellt werden. Die Reaktanden werden in äquimolaren Mengen eingesetzt, wobei zweckmässig ein geringer Überschuss der Verbindung der Formel III verwendet wird.

Als Metalle bzw. Metallverbindungen werden Eisen, Kupfer, Zinn, Cadmium und besonders Zink sowie Zinn(II)chlorid verwendet. Ferner sind auch Mischungen von Metallen oder Metallegierungen geeignet, z. B. Zink/Kupfer, Zink/Quecksilber, Zink/Cadmium und Zink/Blei. Die Metalle, Mischungen und Legierungen können nach Fieser und Fieser, Reagents for Organic Synthesis, Vol. I. John Wiley, N.Y., Seite 1276 (1967) aktiviert werden.

Geeignete inerte organische Lösungsmittel sind bevorzugt polare, besonders polare, aprotische Lösungsmittel, die alleine oder in Mischungen aus mindestens zwei Lösungsmitteln verwendet werden können. Geeignete Lösungsmittel sind zum Beispiel: Ether wie Diethylether, Dibutylether, Tetrahydrofuran, Dioxan, Methylethylenglykol, Dimethylethylenglykol, Dimethyldiethylenglykol, Dieethyldiethylenglykol, Dibutyldiethylenglykol, Dimethyltriethylenglykol, Carbonsäureester und Lactone wie Propylencarbonat, Essigsäureethylester, Propionsäuremethylester, Benzoesäureethylester, Ethylglykolacetat, 2-Methoxyethylacetat, γ-Butyrolacton, γ-Valerolacton und Mevalolacton, Sulfoxide, wie Dimethylsulfoxid, Tetramethylensulfoxid, Sulfone wie Dimethylsulfon, Diethylsulfon, Trimethylensulfon, Tetramethylensulfon, und besonders Säureamide wie z. B. Dimethylformamid, Dimethylacetamid, N-Methylpyrrolidon und Hexamethylphosphorsäuretriamid.

Zur Isolierung der Verbindungen der Formel III wird das Reaktionsgemisch zunächst mit einer Säure, bevorzugt verdünnten wässrigen Säuren wie Salzsäure oder Schwefelsäure behandelt. Hierbei kann man so vorgehen, dass man das Reaktionsgemisch in die verdünnte Säure giesst, wobei die Säure mit Eis vermischt sein kann. Hierauf gibt man ein Lösungsmittel wie z. B. Ether zu und mischt die wässrige und organische Phase zun Herauslösen der Verbindungen. Nach dem Abtrennen der organischen Phase kann die rohe Verbindung durch Verdampfen des Lösungsmittels erhalten werden. Das Rohprodukt kann in üblicher Weise durch Kristallisation, Destillation oder chromatographische Methoden gereinigt werden.

Mit dem erfindungsgemässen Verfahren werden die Verbindungen der Formel II in hoher Ausbeute und Reinheit unter milden Reaktionsbedingungen in einfachen technischen Apparaturen erhalten.

Die Verbindungen der Formel II sind wertvolle Zwischenprodukte zur Herstellung von Wirkstoffen mit $CF_3$-Gruppen im Bereich der Pharmazeutika und der Agrochemikalien. Es wurde ferner gefunden, dass sich die Alkohole und besonders deren Chlorameisensäureester hervorragend als O-, S- oder N-Schutzgruppen insbesondere in der Peptidsynthese eignen. Diese Schutzgruppen sind teilweise unter sehr milden Reaktionsbedingungen abspaltbar. Ferner weisen sie eine erhöhte Lipophilie auf, die zudem durch die Wahl des Restes R in Formel II in einem weiten Bereich variiert werden kann. Durch die erhöhte Lipophilie werden Reaktionen auch in weniger polaren Lösungsmitteln möglich und ferner kann eine spezifische Anpassung der Schutzgruppe an die jeweiligen Reaktionsbedingungen erfolgen.

Ein weiterer Gegenstand vorliegender Erfindung sind Chlorameisensäureester der Formel V

$$F_3CCCl_2\overset{R^3}{\overset{|}{C}}HO-\overset{O}{\overset{\|}{C}}-Cl \qquad (V),$$

worin $R^3$ die in Formel II angegebene Bedeutung hat, sowie deren Verwendung als O-, S- oder N-Schutzgruppe, besonders als N-Schutzgruppe in der Peptidsynthese.

Eine bevorzugte Gruppe von Verbindungen der Formel V sind solche, in denen $R^3$ für ein Wasserstoffatom oder unsubstituiertes oder substituiertes $C_1$-$C_{18}$-Alkyl, $C_2$-$C_{18}$-Alkenyl, Phenyl, $C_7$-$C_{18}$-Alkylphenyl, $C_8$-$C_{18}$-Alkylbenzyl oder Cycloalkyl mit 5 oder 6 Ringkohlenstoffatomen steht.

Die Herstellung der Chlorameisensäureester erfolgt in bekannter Weise durch Umsetzung eines Propanols der Formel II mit Phosgen im molaren Verhältnis von etwa 1:1 bevorzugt bei Raumtemperatur und in Gegenwart einer Base wie Aminen, z. B. Pyridin, und eines inerten Lösungsmittels. Nach beendeter Reaktion behandelt man das Reaktionsgemisch zweckmässig mit einer verdünnten wässrigen Säure und isoliert den Chlorkohlensäureester aus der organischen Phase in üblicher Weise durch Entfernen des Lösungsmittels. Das erhaltene Rohprodukt kann wie zuvor erwähnt gereinigt werden.

Die nachfolgenden Beispiele erläutern die Erfindung näher.

**Beispiele 1 bis 23:**

0,10 Mol Aldehyd und 0,125 g Atom Zinkpulver (aktiviert nach Fieser und Fieser) werden bei Raumtemperatur in 180 ml Dimethylformamid in einem 500 ml Einhalsrundkolben vorgelegt, mit 0,11 Mol 1,1,1-Trichlortrifluorethan versetzt und der Kolben verschlossen. Das Reaktionsgemisch wird in einem Wasserbad während 1 - 60 Stunden kräftig gerührt, wobei nach unterschiedlich langer Zeit eine mässige Exothermieauftritt. Nachdem das Zink in Lösung gegangen ist, wird auf eine Mischung aus 10 %iger wässriger HCl und 300 g Eis gegossen, mit Ether übergossen und während 30 Minuten gerührt. Die Etherphase wird mit 2 %iger wässriger HCl gewaschen, über $MgSO_4$ getrocknet, am Rotationsverdampfer eingeengt und das Produkt je nach Konsistenz destilliert, umkristallisiert oder chromatographiert ($SiO_2/CH_2Cl_2$). Die Ausbeuten liegen zwischen 50 und 100 %. Die Ergebnisse sind in nachfolgender Tabelle zusammengestellt.

**Tabelle**

| Beispiel No. | R-CHO | $CF_3CCl_2CH(OH)R$ | δCH[a)] (ppm) | $δCF_3$[b)] (ppm) | Smp./Sdp.(°) |
|---|---|---|---|---|---|
| | | R = gleich | | | |
| 1 | H | | 4,14 | -77,3 | 61-62 (kristallin) |
| 2 | $CH_2CH_2CH_3$ | | 4,10 | - | 65-67/22mbar |
| 3 | $CH(CH_3)_2$ | | 4,07 | - | 55-60/20mbar |
| 4 | $CH_2O$- FIG1/30 | | | | |
| | | | 4.59 | - | 170/0,01mbar |
| 5 | $CH=CH_2$ | | 4,70 | -75,5 | 58-59/36mbar |
| 6 | $CH=CH-CH_3$ | | 4,59 | -74,9 | 65-67/24mbar |
| 7 | $CH=C(CH_3)_2$ | | 4,85 | -75,2 | 91-93/32mbar |
| 8 | $C(CH_3)=CHCH_2CH_3$ | | 4,59 | - | 88-90/24mbar |
| 9 | $CH=CH-C_6H_5$ | | 4,83 | -74,8 | 90/0,01mbar |
| 10 | Phenyl | | 5,26 | -74,1[c)] | 78/0,01mbar |
| 11 | o-Hydroxyphenyl | | 5,50 | - | gelbes Öl |
| 12 | o-Methoxyphenyl | | 5,62 | -74,6[c)] | 130/0,01mbar |
| 13 | o-Chlorphenyl | | 5,96 | -75,2[c)] | 65-66 (kristallin) |
| 14 | o-Bromphenyl | | 5,98 | - | 61-64 (kristallin) |
| 15 | 2,3-Dichlorphenyl | | 6,00 | - | gelbes Öl |
| 16 | 3-Phenoxyphenyl | | 5,15 | - | 100/0,01mbar |
| 17 | p-Methoxyphenyl | | 5,22 | - | farbloses Öl |
| 18 | p-Bromphenyl | | 5,26 | - | 83-85 (kristallin) |
| 19 | 2,4-Dichlorphenyl | | 5,91 | - | 73-75 (kristallin) |
| 20 | 2,4-Dimethoxyphenyl | | 5,55 | -74,5[c)] | 150/0,01mbar |
| 21 | p-Cyanophenyl | | 5,34 | -74,5[c)] | 60-64 (kristallin) |
| 22 | 1-Furyl | | 5,25 | - | 115/24mbar |
| 23 | 6-Phenoxypyrid-2-yl | | 5,12 | - | 170/0,01mbar |

a) $^{1}H$-NMR($CDCl_3$), relativ zu Tetramethylsilan
b) $^{15}F$-NMR($CDCl_3$) relativ zu $CFCl_3$
c) In $C_6D_6$

**Beispiel 24:**

Gemäss der Vorschrift von Beispiel 1 werden Formaldehyd und 1,1,1-Trichlor-2,2,2-trifluorethan in Dimethylformamid in Gegenwart von Zink unter den nachfolgenden Reaktionsbedingungen zu 1,1,1-Trifluor-2,2-dichlor-propanol umgesetzt:

| | Reaktionszeit | Temperatur | Ultraschall* | Ausbeute |
|---|---|---|---|---|
| 1. | 16 | 20 | - | 46 |
| 2. | 16 | 20 | 38 kHz | 64 |
| 3. | 16 | 20 | 22 kHz | 74 |

*Ultraschallbad der Firma ELGA AG (Biel), Typ B1 14

**Beispiele 25-28:**

Herstellung von Chlorameisensäureestern 0,10 Mol eines 1-substituierten 3,3,3-Trifluor-2,2-dichlorpropanols und 0,11 mol Phosgen werden in 40 ml $CH_2Cl_2$ gelöst und auf -10° abgekühlt. Dann wird 0,10 mol Pyridin in 5 ml

$CH_2Cl_2$ gelöst zugetropft, auf Raumtemperatur erwärmt und über Nacht gerührt. Das Reaktionsgemisch wird auf Eiswasser gegossen und mit $H_2O$ und 10 %iger $H_2SO_4$ aq. nachgewaschen. Nach dem Trocknen der $CH_2Cl_2$-Phase mit MgSO und Einengen am Rotationsverdampfer wird der Rückstand destilliert. Die Ausbeuten liegen zwischen 50 und 100 %. Die Ergebnisse sind in nachfolgender Tabelle angegeben.

| Beispiel No. | $ClCOCHCCl_2CF_3$ (C=O, CH–R) R gleich | δCH[a] (ppm) | $δCF_3$[b] (ppm) | Siedepunkt (°C) |
|---|---|---|---|---|
| 29 | H | 4,80 | 78,0 | 30/20 mbar |
| 30 | $CH_2CH_2CH_3$ | 5,47 | - | 83-86/39 mbar |
| 31 | CH=CH-Phenyl | 5,93 | - | Öl |
| 32 | Phenyl | 6,28 | - | 119-120/30mbar |

**Beispiel 33:**

Die 3,3,3-Trifluor-2,2-dichlorpropyloxycarbonyl (TDPOC)-Schutzgruppe in der Peptidsynthese

a) Herstellung von N-TDPOC-Phe-OH

Unter Schotten-Baumann-Bedingungen werden 0,1 Mol (16,5 g) Phenylalanin, gelöst in 200 ml $H_2O$ und 250 ml 1 N NaOH 100 ml Diethyläther versetzt und bei 0° mit 0,14 Mol (34,4 g) Chlorameisensäure-3,3,3-trifluor-2,2-dichlorpropylester in 140 ml Dioxan 1 Stunde lang emulgiert. Man wäscht die wässrige Phase mit Diethylether, säuert mit 5 N HCl an und extrahiert mit Essigsäureethylester. Nach dem Trocknen mit $MgSO_4$ wird am Rotationsverdampfer eingeengt und der Rückstand über Nacht am Hochvakuum getrocknet. Es resultieren 32,6 g (87 %) N-TDPOC-Phe-OH als gelbliches harziges Öl.

Im $^1H$-NMR (250 MHz; $CDCl_3$) Spektrum erscheint die $CH_2$-Gruppe der TDPOC-Funktion bei 5,36 ppm. Die $CF_3$-Gruppe zeigt im $^{19}F$-NMR ($CDCl_3$) Spektrum ein Signal bei -77,9 ppm ($CCl_3F$ als Standard).

b) Herstellung von N-TDPOC-Phe-Phe-$OC_2H_5$

Die Herstellung des geschützten Dipeptids erfolgt in Analogie zur Vorschrift von J.F. Carson (Synthesis 1981, 268) für die Synthese von N-TROC-Phe-Met-$OC_2H_5$.

0,02 Mol N-TDPOC-Phe-OH gemäss a) werden mit 0,02 Mol $H_2N$-Phe-$OC_2H_5$ mit der DCC/N-Hydroxysuccinimid-Methode kondensiert und ergeben nach Kristallisation aus Ethanol/$H_2O$ 0,017 Mol (85 %) N-TDPOC-Phe-Phe-$OC_2H_5$ vom Smp. 96° C.

Im $^1H$-NMR (250 MHz; $d_6$-DMSO) erscheint die Methylengruppe der TDPOC-Funktion bei 4,65 ppm. Die $CF_3$-Gruppe zeigt im $^{19}F$-NMR ($CDCl_3$) Spektrum ein Signal bei -77,9 ppm ($CCl_3F$ als Standard).

c) Entfernen der TDPOC-Schutzgruppe

Die TDPOC-Gruppe wird unter analogen Bedingungen, wie sie für die TROC-Gruppe in der Literatur beschrieben sind entfernt. (E. Wünsch in Houben Weyl: "Synthese von Peptiden" Bd. I. Herausgeber E. Müller, Georg Thieme Verlag, Stuttgart 1974.)

Der Prozess lässt sich sehr einfach mittels $^1H$-NMR verfolgen, wobei das Verschwinden des TDPOC-$CH^2$-Signals beobachtet wird.

Die folgenden Arbeitsbedingungen ergeben ein Entfernen zum Dipeptid $H_2N$-Phe-Phe-$OC_2H_5$.HCl:

a) 1,0 g (1,8 mMol) des Dipeptids gemäss b) werden in 30 ml EtOH mit 2,0 g Zink während 45 Minuten am Rückfluss erhitzt. Nach Aufarbeitung wird das Produkt in $CHCl_3$ mit HCl (Gas) ins Hydrochlorid überführt, und mit Pentan gefällt. Die Ausbeute an reinem N-ungeschützten Dipeptid beträgt 70 % (0,48 g).

b) Dasselbe Resultat wird erhalten, wenn 1,0 g (1,8 mMol) des Dipeptids während 70 Minuten bei Raumtemperatur mit 2,0 g Zink in Eisessig gerührt wird.

c) Dasselbe Resultat erhält man, wenn 1 g des Dipeptids während 70 Minuten mit 2 g Zink, 2 g $NH_4Cl$ in 40 ml Ethanol bei Raumtemperatur gerührt werden.

**Patentansprüche**

1. Teilhalogenierte α-substituierte 1-Propanole der Formel I

$$\begin{array}{l} \qquad\quad R \\ \qquad\quad | \\ F_3C\text{-}CCl_2\text{-}CH\text{-}OH \end{array} \qquad \text{(I),}$$

worin R ein aliphatischer oder aromatischer Kohlenwasserstoffrest oder ein über ein C-Atom gebundener aliphatischer oder aromatischer heterocyclischer Rest ist, der gegebenenfalls durch $C_1$-$C_{18}$-Alkyl, $C_2$-$C_{18}$-Alkenyl, $C_2$-$C_{18}$-Alkinyl, $C_6$-$C_{18}$-Aryl, $C_7$-$C_{18}$-Aralkyl, $C_7$-$C_{18}$-Alkaryl, $C_8$-$C_{18}$-Alkaralkyl, $C_8$-$C_{18}$-Alkoxy, $C_1$-$C_{18}$ -Alkylthio, $C_6$-$C_{18}$-Aryloxy, $C_7$-$C_{18}$-Aralkoxy, $C_7$-$C_{18}$-Alkaryloxy, $C_8$-$C_{18}$-Alkaralkoxy, $C_7$-$C_{18}$-Aryloxyalkyl, $C_7$-$C_{18}$-Alkoxyaryl, $C_3$-$C_{12}$-Cycloalkyl, $C_3$-$C_{12}$-Cycloalkoxy, $C_3$-$C_{12}$-Cycloalkenyl, $C_3$-$C_{12}$-Cycloalkenyloxy, -COOH, $-CONH_2$, F, -CN, -OH, -SH, $-NH_2$, -COOR, -CONHR, $-CONR^1R^2$, $-NHR^1$, $-NR^1R^2$, worin $R^1$ $C_1$-$C_{18}$-Alkyl, Cyclohexyl, Phenyl oder Benzyl ist und $R^2$ unabhängig die gleiche Bedeutung wie $R^1$ hat, für R in der Bedeutung als aromatischer Rest auch durch Br oder Cl substituiert ist, und wobei R auch p-Chlorphenoxymethyl sein kann oder in der Bedeutung als aliphatischer Rest gegebenenfalls durch -CO- unterbrochen ist.

2. Propanole gemäss Anspruch 1, dadurch gekennzeichnet, dass R als Kohlenwasserstoffrest 1 bis 30 C-Atome und als heterocyclischer Rest 1 bis 3 Heteroatome aus der Gruppe O, N und S im Ring enthält.

3. Propanole gemäss Anspruch 1, dadurch gekennzeichnet, dass R 1- bis 3-fach substituiert ist.

4. Propanole gemäss Anspruch 1, dadurch gekennzeichnet, dass R als aliphatischer Kohlenwasserstoffrest Alkyl, Alkenyl, Alkinyl, Cycloalkyl oder Cycloalkenyl, als aromatischer Kohlenwasserstoffrest Aryl, Aralkenyl, Aralkinyl oder Aralkyl, als heterocyclisch-aliphatischer Rest ein 4- bis 6-gliedriger, 1 oder 2 Heteroatome aus der Gruppe O, N und S enthaltender heterocyclischer Rest und als aromatisch-heterocyclischer Rest ein 5- oder 6-gliedriger, 1 oder 2 Heteroatome aus der Gruppe O, N oder S enthaltender heterocyclischer Rest ist, der gegebenenfalls wie in Anspruch 1 definiert substituiert ist.

5. Verfahren zur Herstellung von teilhalogenierten α-substituierten 1-Propanolen der Fornel II

$$\begin{array}{l} \qquad\quad R^3 \\ \qquad\quad | \\ F_3CCCl_2\text{-}CH\text{-}OH \end{array} \qquad \text{(II),}$$

worin R für ein Wasserstoffatom steht oder die gleiche Bedeutung wie R in Formel 1 gemäss Anspruch 1 hat, dadurch gekenzeichnet, dass man 1,1,1-Trifluor-2,2,2-trichlorethan der Formel III

$$F_3C\text{-}CCl_3 \qquad \text{(III),}$$

mit einem Aldehyd der Formel IV

$$R^3\text{-}CHO \qquad \text{(IV),}$$

worin $R^3$ die für Formel II angegebene Bedeutung hat, in Gegenwart eines Metalles, Mischungen von Metallen oder Metallegierungen aus der Gruppe Eisen, Kupfer, Zinn, Cadmium und Zink, oder der Metallverbindung Zinn(II)chlorid, und in Gegenwart eines inerten Lösungsmittels umsetzt, das Reaktionsgemisch nach Beendigung der Reaktion mit einer Säure behandelt und danach das 1-Propanol der Formel II in an sich bekannter Weise isoliert.

6. Verfahren gemäss Anspruch 5, dadurch gekennzeichnet, dass die Reaktion bei Raumtemperatur durchgeführt wird.

7. Verfahren gemäss Anspruch 5, dadurch gekennzeichnet, dass die Umsetzung unter Einwirkung von Ultraschall vorgenommen wird.

8. Verfahren gemäss Anspruch 5, dadurch gekennzeichnet, dass das Lösungsmittel ein polares aprotisches Lösungsmittel ist.

9. Verfahren gemäss Anspruch 5, dadurch gekennzeichnet, dass man das Reaktionsgemisch mit einer verdünnten wässrigen Säure behandelt.

10. Verfahren gemäss Anspruch 8, dadurch gekennzeichnet, dass das Lösungsmittel Dimethylformamid, Dimethylacetamid, N-Methylpyrrolidon oder Hexamethylphosphorsäuretriamid ist.

11. Chlorkohlensäureester der Formel V

$$\begin{array}{l} \qquad\;\; R^3 \quad\; O \\ \qquad\;\; | \qquad \| \\ F_3CCCl_2CHO\text{-}C\text{-}Cl \end{array} \qquad \text{(V),}$$

worin $R^3$ die in Anspruch 5 angegebene Bedeutung hat.

12. Verwendung der Chlorkohlensäureester gemäss Anspruch II als O-, S- oder N-Schutzgruppe, insbesondere als N-Schutzgruppe in der Peptidsynthese.

**Claims**

1. A partially halogenated, α-substituted 1-propanol of formula I

$$\begin{array}{l} \quad\quad\quad\; R \\ \quad\quad\quad\; | \\ F_3C\text{-}CCl_2\text{-}CH\text{-}OH \end{array} \qquad (I),$$

wherein R is an aliphatic or aromatic hydrocarbon radical or an aliphatic or aromatic heterocyclic radical bound through a C atom, each of which radicals is unsubstituted or substituted by $C_1$-$C_{18}$alkyl, $C_2$-$C_{18}$alkenyl, $C_2$-$C_{18}$alkynyl, $C_6$-$C_{18}$aryl, $C_7$-$C_{18}$aralkyl, $C_7$-$C_{18}$alkaryl, $C_8$-$C_{18}$alkaralkyl, $C_1$-$C_{18}$alkoxy, $C_1$-$C_{18}$alkylthio, $C_6$-$C_{18}$aryloxy, $C_7$-$C_{18}$aralkoxy, $C_7$-$C_{18}$alkaryloxy, $C_8$-$C_{18}$alkaralkoxy, $C_7$-$C_{18}$aryloxyalkyl, $C_7$-$C_{18}$alkoxyaryl, $C_3$-$C_{12}$cycloalkyl, $C_3$-$C_{12}$cycloalkoxy, $C_3$-$C_{12}$cycloalkenyl, $C_3$-$C_{12}$cycloalkenyloxy, -COOH, $-CONH_2$, F, -CN, -OH, -SH, $-NH_2$, $-COOR^1$, $-CONHR^1$, $-CONR^1R^2$, $-NHR^1$ or $-NR^1R^2$, wherein $R^1$ is $C_1$-$C_{18}$alkyl, cyclohexyl, phenyl or benzyl, and $R^2$ independently has the same meaning as $R^1$; and R as an aromatic radical may also be substituted by Br or Cl, and R may also be p-chlorophenoxymethyl, or as an aliphatic radical may be interrupted by -CO-.

2. A propanol according to claim 1, wherein R as a hydrocarbon radical contains 1 to 30 carbon atoms and, as a heterocyclic radical, contains in the ring 1 to 3 hetero atoms selected from the group O, N and S.

3. A propanol accordimg to claim 1, wherein R carries 1 to 3 substituents.

4. A propanol according to claim 1, wherein R as an aliphatic hydrocarbon radical is alkyl, alkenyl, alkynyl, cycloalkyl or cycloalkenyl, and as an aromatic hydrocarbon radical is aryl, aralkenyl, aralkynyl or aralkyl and R as heterocyclic-aliphatic radical is a 4- to 6-membered heterocyclic radical which contains 1 or 2 hetero atoms, and as an aromatic-heterocyclic radical is a 5- or 6-membered heterocyclic radical which contains 1 or 2 hetero atoms selected from the group O, N and S, each of which radicals is unsubstituted or substituted as defined in claim 1.

5. A process for the preparation of a partially halogenated, α-substituted 1-propanol of formula II

$$\begin{array}{l} \quad\quad\quad\; R^3 \\ \quad\quad\quad\; | \\ F_3CCCl_2\text{-}CH\text{-}OH \end{array} \qquad (II),$$

wherein $R^3$ is a hydrogen atom or has the same meaning as R in formula I according to claim 1, which process comprises reacting 1,1,1-trifluoro-2,2,2-trichloroethane of formula III

$$F_3C\text{-}CCl_3 \qquad (III),$$

with an aldehyde of formula IV

$$R^3\text{-}CHO \qquad (IV),$$

wherein $R^3$ is as defined for formula II, in the presence of a metal, a mixture of metals or metal alloys selected from the group iron, copper, tin, cadmium and zinc, or of the metal compound tin(II) chloride, and in the presence of an inert solvent, treating the reaction mixture with an acid upon completion of the reaction, and subsequently isolating the 1-propanol of formula II in a manner known per se.

6. A process according to claim 5, wherein the reaction is carried out at room temperature.

7. A process according to claim 5, wherein the reaction is carried out with ultrasonics.

8. A process according to claim 5, wherein the solvent is a polar aprotic solvent.

9. A process according to claim 5, wherein the reaction mixture is treated with a dilute aqueous acid.

10. A process according to claim 8, wherein the solvent is dimethylformamide, dimethylacetamide, N-methylpyrrolidone or hexamethylphosphoric triamide.

11. A chloroformate of formula V

$$\begin{array}{l} \quad\quad\quad R^3 \quad\; O \\ \quad\quad\quad | \quad\;\; \| \\ F_3CCCl_2\text{-}CHO\text{-}C\text{-}Cl \end{array} \qquad (V),$$

wherein $R^3$ is as defined in claim 5.

12. Use of a chloroformate according to claim 11 as -O-, -S- or -N- protective group in peptide synthesis.

**Revendications**

1. Propanols-1 partiellement halogénés et substitués en α qui répondent à la formule I:

$$F_3C\text{-}CCl_2\text{-}CH(R)\text{-}OH \qquad (I)$$

dans laquelle R représente un radical hydrocarboné aliphatique ou aromatique ou un radical hétérocyclique aliphatique ou aromatique lié par un de ses atomes de carbone, qui porte éventuellement un alkyle en $C_1$-$C_{18}$, un alcényle en $C_2$-$C_{18}$, un alcynyle en $C_2$-$C_{18}$, un aryle en $C_6$-$C_{18}$, un aralkyle en $C_7$-$C_{18}$, un alkylaryle en $C_7$-$C_{18}$, un alkylaralkyle en $C_8$-$C_{18}$, un alcoxy en $C_1$-$C_{18}$, un alkylthio en $C_1$-$C_{18}$, un aryloxy en $C_6$-$C_{18}$, un aralcoxy en $C_7$-$C_{18}$, un alkylaryloxy en $C_7$-$C_{18}$, un alkylaralcoxy en $C_8$-$C_{18}$, un aryloxyalkyle en $C_7$-$C_{18}$, un alcoxyaryle en $C_7$-$C_{18}$, un cycloalkyle en $C_3$-$C_{12}$, un cycloalcoxy en $C_3$-$C_{12}$, un cycloalcényle en $C_3$-$C_{12}$ un cycloalcényloxy en $C_3$-$C_{12}$, un radical -COOH, $-CONH_2$, F, -CN, -OH, -SH ou $-NH_2$, un radical $-COOR^1$, un radical $-CONHR^1$, un radical $-CONR^1R^2$, un radical $-NHR^1$ ou un radical $-NR^1R^2$ dans lesquels $R^1$ et $R^2$ représentent chacun, indépendamment l'un de l'autre, un alkyle en $C_1$-$C_{18}$ un cyclohexyle, un phényle ou un benzyle, un radical aromatique R pouvant également porter Br ou Cl, et R pouvant être aussi un radical chloro-4 phénoxyméthyle ou pouvant, lorsqu'il désigne un radical aliphatique, être interrompu par un radical -CO-.

2. Propanols selon la revendication 1 caractérisés en ce que R, en tant que radical hydrocarboné, contient de 1 à 30 atomes de carbone et, en tant que radical hétérocyclique, contient dans son cycle de 1 à 3 hétéroatomes pris dans l'ensemble constitué par O, N et S.

3. Propanols selon la revendication 1 caractérisés en ce que R porte de 1 à 3 substituants.

4. Propanols selon la revendication 1 caractérisés en ce que R est, en tant que radical hydrocarboné aliphatique, un radical alkyle, alcényle, alcynyle, cycloalkyle ou cycloalcényle, en tant que radical hydrocarboné aromatique un radical aryle, aralcényle, aralcynyle ou aralkyle, en tant que radical aliphatique hétérocyclique un radical hétérocyclique comportant de 4 à 6 maillons et contenant 1 ou 2 hétéroatomes pris dans l'ensemble constitué par O, N et S, et, en tant que radical hétérocyclique aromatique, un radical hétérocyclique comportant 5 ou 6 maillons et contenant 1 ou 2 hétéroatomes pris dans l'ensemble constitué par O, N et S, radical qui est éventuellement substitué comme indiqué à la revendication 1.

5. Procédé de préparation de propanols-1 partiellement halogénés et substitués en α qui répondent à la formule II:

$$F_3CCCl_2\text{-}CH(R^3)\text{-}OH \qquad (II)$$

dans laquelle $R^3$ représente un atome d'hydrogène ou a la signification qui a été donnée pour le symbole R de la formule I à la revendication 1, procédé caractérisé en ce qu'on fait réagir le trifluoro-1,1,1 trichloro-2,2,2 éthane de formule III:

$$F_3C\text{-}CCl_3 \qquad (III)$$

avec un aldéhyde répondant à la formule IV:

$$R^3\text{-}CHO \qquad (IV)$$

dans laquelle $R^3$ a la signification qui lui a été donnée à propos de la formule II, en présence d'un métal, de mélanges de métaux ou d'alliages de métaux pris dans l'ensemble constitué par le fer, le cuivre, l'étain, le cadmium et le zinc, ou du composé métallique qu'est le chlorure d'étain (II), et en présence d'un solvant inerte, on traite le mélange réactionnel, après la fin de la réaction, par un acide, et ensuite on isole de manière connue le propanol-1 de formule II.

6. Procédé selon la revendication 5 caractérisé en ce qu'on effectue la réaction à la température ambiante.

7. Procédé selon la revendication 5 caractérisé en ce qu'on effectue la réaction sous l'action d'un ultrason.

8. Procédé selon la revendication 5 caractérisé en ce que le solvant est un solvant aprotique polaire.

9. Procédé selon la revendication 5 caractérisé en ce qu'on traite le mélange réactionnel par un acide aqueux dilué.

10. Procédé selon la revendication 8 caractérisé en ce que le solvant est le diméthylformamide, le diméthylacétamide, la N-méthylpyrrolidone ou l'hexaméthylphosphorotriamide.

11. Esters de l'acide chloroformique qui répondent à la formule V:

$$F_3CCCl_2CH(R_3)O\text{-}C(=O)\text{-}Cl \qquad (V)$$

dans laquelle $R^3$ a la signification donnée à la revendication 5.

12. Application des esters chloroformiques selon la revendication 11 pour la création de radicaux protecteurs de O, de S ou de N, plus particuliérement de radicaux protecteurs de N dans la synthèse de peptides.